# EUROPEAN PATENT SPECIFICATION

(11) **EP 2 220 004 B1**
(45) Date of publication and mention of the grant of the patent: **26.09.2012**
(21) Application number: 08856133.7
(22) Date of filing: 05.12.2008
(51) Int. Cl.: C02F 11/04, C02F 3/28, C02F 1/461

(54) **METHOD AND DEVICE FOR CONVERTING BIOMASS INTO METHANE**
VERFAHREN UND VORRICHTUNG ZUR UMWANDLUNG VON BIOMASSE IN METHAN
PROCÉDÉ ET DISPOSITIF DE CONVERSION DE LA BIOMASSE EN MÉTHANE

(30) Priority: 07.12.2007 NL 2001067
(43) Date of publication of application: 25.08.2010
(73) Proprietor: Spark Origin B.v., 6511 ZD Nijmegen (NL)
(72) Inventor: GERRITSEN, Jan Willem, NL-6602 EP Wijchen (NL); BLANKENBORG, Stephanus Gerardus Johannes, NL-6511 ZD Nijmegen (NL)
(74) Representative: Langenhuijsen, Bastiaan Wilhelmus Herman
(86) International application number: PCT/NL2008/050779
(87) International publication number: WO 2009/072887

(56) References cited:
- US-A- 5 630 942
- US-A1- 2005 194 311

## Description

The invention relates to a method for converting biomass into methane. The invention also relates to a device for converting biomass into methane, in particular by making use of the method according to the invention.

The use of fossil fuels has shown a very considerable increase in the last 100 years. Calculations indicate that, with the present increasing consumption of fossil fuels, reserves of coal can meet consumer demand for about another 200 years, natural gas reserves for between 70 and 100 years and oil reserves for between only 40 and 50 years. In addition to the fact that the reserves of fossil fuels are relatively limited, the combustion of the fossil fuels involves the production of carbon dioxide, and this results in increased global warming. Different initiatives are therefore being initiated to obtain renewable energy in order to be able to provide the human race with energy for an unlimited period of time without damaging the environment and the prospects for future generations. Examples of renewable energy are wind energy, solar energy and energy from biomass. The drawback of wind energy and solar energy relative to energy obtained from biomass is however that these types of energy can produce only electricity or heat, but not fuel, at least not directly. Since the world will still depend for a long time to come on fuels, for instance for traffic and transport, heating and decentralized energy supply, there is an increasing need for an efficient conversion of biomass into fuel, including methane (CH₄). Energy can be obtained from this biomass by burning, gasifying, fermenting or pyrolysing the biomass. According to European Directive 2001/77/EC, biomass is defined as the biologically degradable fraction of products, waste and residues from agriculture (including vegetal and animal substances), forestry and related industries, as well as the degradable fraction of industrial and municipal waste. In an alternative definition biomass is deemed to be a collective name for all the material (mass) produced by living organisms, including wood, specially cultured crops, organic-biological vegetable or animal waste, manure and sludge from wastewater treatment plants. The use of specially cultured energy-rich crops, such as for instance rape seed, sugarcane and maize, is however not generally compatible with the increasing world food shortage. There are in practice diverse biomass-related alternatives to the use of energy-rich crops which will not create a conflict between the increasing energy demand and the increasing shortage of food.

Although the existing potential of biological-organic waste is acknowledged, it has not yet been found readily possible to obtain commercially feasible (green) fuel from these (green) waste flows in effective manner. The reason for these problems is that the energy still present in these biological-organic waste flows is stored in the form of complex biomolecules such as for instance cellulose, proteins and facts, which are relatively difficult to convert chemically to biological fuels. This chemical conversion is further impeded by the relatively large quantity of water present in this biomass. The biochemical conversion of this biomass into biological fuel can be accelerated by catalyzing the conversion using enzymes. These enzymes are however expensive and cannot always withstand the environment in which the biochemical conversion has to take place. It is also known to apply micro-organisms instead of enzymes to enable fermentation of the biomass to methane (natural gas). This microbiological conversion process takes place in the presence of oxygen, whereby the greatest possible energy-saving is ensured. A significant drawback of this microbiological conversion is that the biomass must be situated in a bioreactor for a relatively long period of time, generally a period of between three and four weeks, to enable the conversion of a fraction of the biomass into methane, and this results in a relatively low efficiency per unit of time. Because the existing process is relatively slow, it moreover involves relatively high investment costs and operational costs. There is a need to improve the microbiological conversion of biomass into methane in order to be able to meet the needs of the increasing global energy requirement. An example of a prior art process and device is given in the document US 2005 0194311.

The invention has for its object to provide a relatively efficient method for the microbiological conversion of biomass into methane.

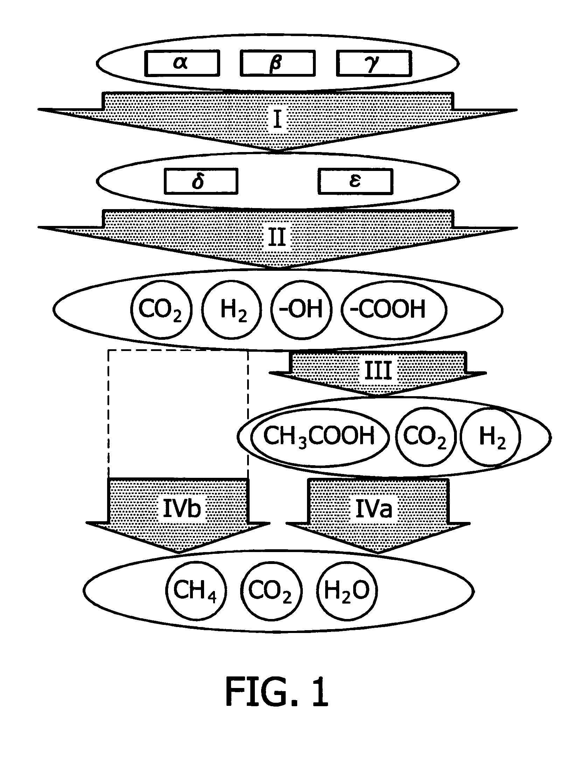

The invention provides for this purpose a method of the type stated in the preamble, comprising the steps of: A) fermenting at least a part of the biomass to form at least one intermediate product, in particular at least one intermediate product chosen from the group of: hydrogen, carbon dioxide and acetate, carbon monoxide, volatile fatty acids, formate, alcohols, pyruvate, methylamine, dimethylamine, methyl sulphide, dimethyl sulphide, trimethylamine, methyl mercaptan, and incompletely reduced carbon compounds, and B) fermenting at least a part of the at least one intermediate product as according to step A) to form at least methane, wherein the method also comprises the following steps of: C) generating at least one base and at least one acid using at least one electrochemical cell, D) adding at least one base to the biomass during step A), and E) adding at least one acid to the intermediate product during step B). By allowing phased fermentation by anaerobic micro-organisms of the biomass generally provided with moisture, wherein in a first phase base is added to the (wet) biomass to be converted into one or more intermediate products, and wherein in a subsequent second phase acid is added to the at least one intermediate product to be converted into methane, the conversion of biomass into methane can be accelerated substantially from three to four weeks to a few days. The advantageous effect of successively adding base and acid to respectively the biomass and the at least one intermediate product can be explained by examining the biochemical conversion of the biomass in detail, as will be set forth hereinbelow. Biomass generally comprises a considerable fraction of undissolved, biodegradable polymers such as proteins, carbohydrates and fats. In a first step these polymers will be converted by means of hydrolysis (I) into less complex dissolved compounds, such as amino acids, (higher) fatty acids, sugars and alcohols by usually extracellular enzymes produced by fermentative micro-organisms. The dissolved compounds are converted by the fermentative micro-organisms into a series of simple compounds such as volatile fatty acids, alcohols, lactic acid, carbon dioxide, hydrogen (H₂), ammonia and dihydrogen sulphide, wherein the micro-organisms create new cell material using the released energy and a part of the formed products. This process is also generally referred to as fermentation or acidogenesis (II). A relatively large part of the fermentation products, generally about 70%, is then converted in a subsequent acetogenesis (III) by anaerobic acetogenic micro-organisms into intermediate products mainly formed by acetate, hydrogen and carbon dioxide, wherein new cell material is also formed. In addition to (or instead of) the above stated intermediate products, carbon dioxide, volatile fatty acids, formate, alcohols, pyruvate, methylamine, dimethylamine, methyl sulphide, dimethyl sulphide, trimethylamine, methyl mercaptan, incompletely reduced carbon compounds or other compounds can also be formed as intermediate product, although these intermediate products will generally be formed only in limited quantities. Where the intermediate products acetate, carbon dioxide and hydrogen are referred to hereinbelow, the above stated alternative intermediate products are also included for the purpose of enabling conversion of biomass into methane. The formed intermediate products are then converted in a methanogenesis (IV) - a methane-forming process - into carbon dioxide and methane by anaerobic methanogenic micro-organisms. A remaining, relatively small part, generally about 30%, of the fermentation products will not be converted into the above stated intermediate products by means of acetogenesis, but will be converted into mainly carbon dioxide and methane directly by means of methanogenesis. The overall fermentation process is also shown schematically in figure 1.

The fermentative micro-organisms, such as for instance Clostridium genera and Bacteroides genera are acid-producing micro-organisms. In order to be able to guarantee sufficient activity of the fermentative and acetogenic micro-organisms it is important to prevent the pH falling below a critical pH value of 5 by adding base as according to step D). The addition of base is also important to keep the pH sufficiently high for the methanogenic micro-organisms which will then in accordance with B) convert the one or more intermediate products into methane. This is because these methanogenic micro-organisms display hardly any activity in a relatively acidic environment. The addition of at least one base to the biomass for fermenting so as to enable an increase in the pH thus has advantages for the most relevant micro-organisms which play a part in the conversion of biomass into methane.

During the methanogenesis acid is added to the intermediate products as according to step E) since the methanogenic micro-organisms display acid-consuming behaviour, and thus need acid in order to be able to achieve an effective biochemical conversion to methane. Both the acetotrophic methanogens, which convert acetate into methane and carbon dioxide, and the hydrogenotrophic methanogens, which convert carbon dioxide and hydrogen (H₂) into methane and water, consume protons (H⁺) in order to realize the biochemical conversion. The methanogenesis can be illustrated by means of the following reaction equations:
Methane formation from hydrogen/carbon dioxide: 4 H₂ + HCO₃⁻ + H⁺ → CH₄ + 3 H₂O
Methane formation from acetate: CH₃COO⁻ + H⁺ → CO₂ + CH₄

Other than the base added during step A), the acid is not added during step B) primarily to regulate the pH of the environment in which the intermediate products are situated, but rather to enable sufficient starting product to be provided in order to obtain the most efficient possible conversion of intermediate product into methane. Successive administering of a base and an acid also has the important advantage that no buffer need be added to the biomass, which would result in an (undesirable) increase in the salt concentration in the biomass. The use of a base and acid instead of a buffer is moreover advantageous from a financial and logistical viewpoint. It is also the case here that, by applying a base and an acid instead of a buffer, a substantially improved process control can be realized because of the successive base requirement and acid requirement during the phased conversion of biomass to methane. It is noted that the conversion of the biomass into the at least one intermediate product and the conversion of the at least one intermediate product into methane generally also take place simultaneously. During step A) biomass will however be mainly converted into the at least one intermediate product, wherein a small fraction of the intermediate product is optionally converted into methane, and during step B) the at least one intermediate product will mainly be converted into methane, wherein a further residual fraction of the original biomass is optionally converted into the at least one intermediate product. Acetate, carbon dioxide and hydrogen will in practice generally be formed as intermediate product. Where in the present description or claims the term biomass is used, this will in practice be a mixture of biomass and intermediate products, wherein the main fraction is formed by biomass. Where in the present description or claims the term intermediate product is used, this will in practice also be a mixture of biomass and intermediate products, wherein the main fraction is formed by one or more intermediate products. Although the process according to the invention is substantially of anaerobic nature, the biomass for converting will however generally be provided initially with oxygen. This oxygen will be consumed relatively quickly during the process by the micro-organisms. The anaerobic conversion process will take place in the meantime (or possibly thereafter).

The base to be administered during step A) can be of diverse nature and, as already stated, will only play the part of proton acceptor in order to prevent a fall in the pH of the biomass below a critical limit of 5. Examples of applicable bases are: OH⁻, S²⁻, PO₄³⁻, NH₃ etc. An additional amount of base can optionally be administered to the at least one intermediate product just before step B) is performed in order to prevent the pH becoming too low during step B) for (sufficiently rapid) methanogenesis. The acid to be administered during step B) is preferably formed by a proton (H⁺) which will be bonded to a water molecule with formation of H₃O⁺. The proton can here be dissociated from a (strong) acid, such as for instance HNO₃, although it is also possible to envisage the proton being dissociated from a weak acid, such as for instance acetic acid. The acid can also be formed by one of the intermediate products formed during step A). The base or acid formed during the biochemical conversion of biomass into methane can, as supplement to the base and acid produced using the at least one electrochemical cell, thus also be usefully employed to further optimize the biochemical conversion. An additional advantage of applying a plurality of base-generating sources and a plurality of acid-generating sources is that the capacity of the electrochemical cell can be reduced, this generally also being advantageous from a commercial viewpoint. In order to enable the acid balance to be kept as neutral as possible it can be advantageous, depending on the situation, for the (effective) molar quantities of the base supplied during step A) and the acid supplied during step B) to be substantially identical, so that during the conversion of biomass to methane the same amount of administered base is neutralized as is subsequently consumed of the administered acid. In determined situations, wherein for instance relatively acidic (or basic) biomass is taken as starting material, it may however be advantageous to administer more (or less) base than acid is successively administered.

The generating of the base and the acid preferably takes place at the location where the biomass and the intermediate product are converted. The generating of the base and the acid according to step C) takes place at least using at least one electrochemical cell which preferably comprises two mutually coupled electrochemical half cells, this considerably enhancing the efficiency of the method for converting biomass to methane according to the invention since the required acid and the required base can be manufactured relatively quickly, easily and inexpensively at the location of the conversion of the biomass. In an electrochemical cell there occurs a redox reaction wherein electrical energy is used. The electrochemical cell comprises two mutually connected electrodes, wherein a reduction reaction takes place at the cathode while an oxidation reaction takes place at the anode. In the electrochemical cell applied in this preferred embodiment the chemical compounds to be converted are chosen such that a base is formed at the cathode while an acid is formed at the anode. When the chemical compositions in the separate half cells differ from each other, these half cells will generally have to be separated, for instance by means of a conductive salt bridge or a conductive membrane, whereby the electrochemical cell is in fact divided into two half cells. Having a base and an acid generated via a redox reaction is relatively advantageous since the base and the acid can be generated on site from one or more (inexpensive) bulk substances. In a particular preferred embodiment the compound for converting is the same substance, in particular water. In order to allow a redox reaction to occur at the electrodes it is however desirable in that case that a power source, such as for instance a battery or accumulator, is coupled to the electrodes in order to actively initiate and sustain the redox reaction. Causing a compound to react, in particular decompose, by applying electric current is also referred to as electrolysis. The advantages of applying water is that water is relatively inexpensive, a typical bulk substance and non-hazardous. Furthermore, water will decompose during the electrolysis into OH⁻ and H⁺ in accordance with the reaction diagram below:
Cathode: 4 H₂O + 4e⁻ → 4 OH⁻ + 2 H₂
Anode: 2 H₂O → O₂ + 4 H⁺ + 4 e⁻
Net: 6 H₂O → 4 OH⁻ + 4H⁺ + 2H₂ + O₂

A base (OH⁻) as well as an acid (H⁺) are thus formed during the electrolysis of water. The same molar amount of base and acid is moreover formed during the electrolysis of water, whereby the administering of the base and the acid during the conversion of biomass to methane does not in principle result in an overall change in the acidity, whereby an unchanged acidity can be guaranteed. In order to prevent as far as possible regeneration of water (H₂O) on the basis of the formed based (OH⁻) and the formed acid (H⁺), a conductive barrier, such as for instance a membrane, is preferably placed between the electrodes in the water. The use of two electrochemical half cells which are mutually coupled by a conductive salt bridge and each filled with water is also possible to enable the best possible mutual separation, and therefore storage, of the formed base and the formed acid. The likewise formed oxygen gas (O₂) and hydrogen gas (H₂) can optionally be collected and subsequently employed usefully. It is for instance possible here to envisage supplying the formed gas to a desulphurization plant which removes the corrosive H₂S, present in small quantities, from the formed biogas.

It can be advantageous when the generating of the base according to step C) additionally takes place in the generally wet biomass, and preferably also that the generating of the acid according to step C) additionally takes place in the generally also wet intermediate product, whereby a separate electrochemical cell positioned at a distance from the biomass and intermediate product(s) to be converted is not required, which will in general considerably simplify structurally the device for converting biomass to methane.

However, in another preferred embodiment the generating of the base and the acid according to step C) takes place at a distance from (although usually in the vicinity of) the biomass and the intermediate product. This preferred embodiment has the advantage that the electrolysis process is made more readily manageable, wherein the formed oxygen gas and the hydrogen gas, which together form a flammable mixture, can moreover be collected in facilitated manner, which will generally be advantageous from the safety viewpoint. Furthermore, the forming of oxygen in the biomass or the intermediate product is generally less advantageous for the activity of the micro-organisms playing a part in the conversion of biomass to methane.

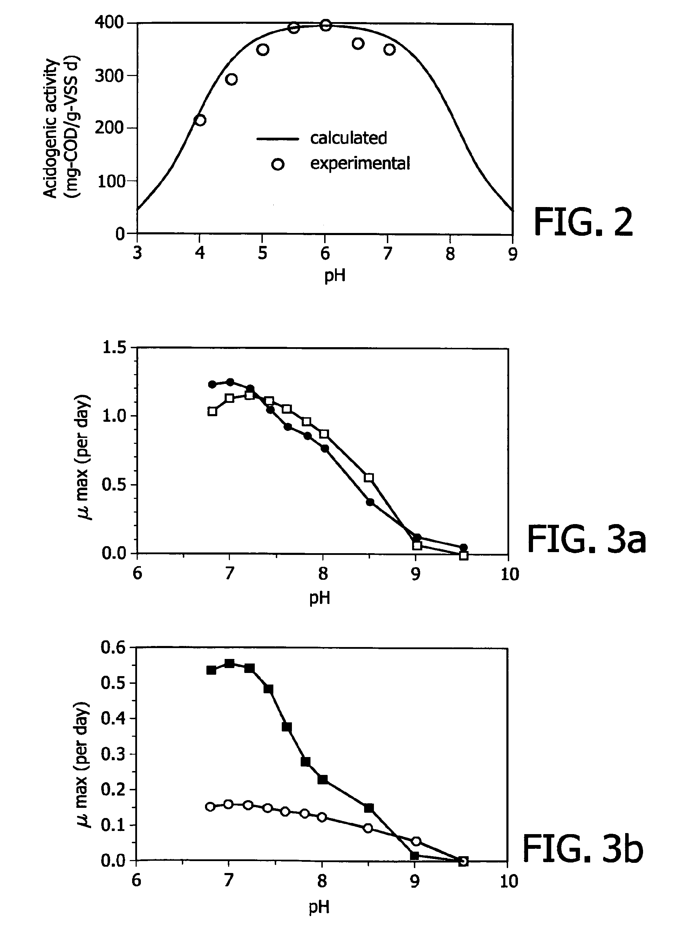

The pH during step A) preferably lies between 5 and 7. The hydrolysis, the acidogenesis and (a part of) the acetogenesis mainly take place during step A). As stated above, fermentative micro-organisms are responsible for the hydrolysis and the acidogenesis. Examples of fermentative micro-organisms responsible for the hydrolysis are micro-organisms belonging to one of the following groups: Clostridium and Ruminococcus. Examples of the micro-organisms responsible for the acidogenesis are micro-organisms belonging to one of the following groups: Clostridium, Fusobacterium, Eubacterium and Propionibacterium. The above stated genera have optimum activity at a pH of between 5 and 7, which is also shown in figure 2, in which the overall acidogenic activity of these microorganisms (in wastewater) is shown as a function of the pH. When the pH becomes lower than 5 or higher than 7, the biological activity of these micro-organisms will then decrease relatively quickly. The fall in acidity which generally accompanies the acidogenesis will be countered by the added base. The method preferably also comprises step F) of detecting the pH of the biomass during step A). In a particular preferred embodiment the quantity of base to be administered as according to step D) is at least determined on the basis of the pH detected during step F). The pH can be held within the above stated range by detecting the pH during step A). It is moreover also possible in this way to detect whether, at a determined moment, there will be a reduced acid production or even a (substantial) rise in pH, this indicating a decrease in the acid production of the micro-organisms, which shows that a substantial part of the biomass has been converted into carbon dioxide, hydrogen and/or acetate.

During step B) mainly the intermediate products are converted into methane by means of methanogenesis. It will optionally also be possible for acetogenesis to take place to some extent during step B). In addition, hydrolysis and acidogenesis optionally also take place during step B) if they have not been completed during step A). Methanogenic micro-organisms are responsible for the methanogenesis. Examples of methanogenic micro-organisms are micro-organisms belonging to one of the following groups: Methanobacterium, Methanobrevibacter, Methanosphaera, Methanothermus, Methanococcus, Methanomicrobium, Methanogenium, Methanospirillium, Methanoplanus, Methanocorpusculum, Methanoculleus, Methanosarcina, Methanolobus, Methanohalobium, Methanococcoides, Methanothrix and Methanopyrus. The above stated acid-consuming micro-organisms generally flourish optimally in a climate with a pH of between 6.5 and 8.0, as also shown in figures 3a and 3b in which the methanogenic activity of different micro-organisms is shown. In a preferred embodiment the method also comprises step G), comprising of detecting the pH of the intermediate product during step B). It is also possible in this way to detect the moment at which a decrease in acid consumption, or even a (substantial) fall in pH occurs, this indicating a decline in the biological activity of the methanogenic micro-organisms, which shows that a substantial part of the intermediate products has been converted into methane. It is also possible that the pH has become so high due to formation of methane that this inhibits further methanogenesis. The quantity of acid to be administered as according to step E) is preferably determined at least on the basis of the pH detected during step G), and in particular a detected (substantially) reduced acid requirement and/or a detected (substantial) fall in pH.

In a preferred embodiment the method also comprises the following steps of: H) guiding the biomass into a first compartment prior to fermenting of at least a part of the biomass according to step A), and I) displacing at least a part of the intermediate product from the first compartment to a second compartment prior to fermenting of at least a part of the intermediate product according to step B). The first compartment and the second compartment in fact form bioreactors in which the fermentation of biomass to methane can be successively realized. It is possible here to envisage the first compartment and the second compartment being mutually integrated to form one compartment, into which the base and the acid are successively guided. After having converted sufficient biomass to methane, which can for instance be determined by means of pH measurements as stated above, the compartment will be largely emptied. A relatively small material fraction, generally in the order of magnitude of 5 to 10%, will remain in the compartment to serve as growth substrate for new micro-organisms for the purpose of enabling conversion of biomass freshly introduced into the compartment. However, since it takes a certain time before the concentration of micro-organisms is sufficiently large that this is no longer the limiting factor for the speed of the conversion process, it is important to select as carefully as possible the size of the material fraction to be left behind. The concentration of micro-organisms in a bioreactor grows as a function of time typically according to a curve which in the first instance rises relatively gradually, subsequently rises sharply (almost exponentially) and then gradually rises further to a saturation level. After a relatively slow start because of a relatively low initial concentration of micro-organisms, possibly combined with non-optimal growth conditions - determined particularly by the pH, chemical composition and temperature - the number of micro-organisms very quickly increases exponentially and then continues to grow in very slow manner to a more or less constant value. Further growth can be inhibited by for instance a limited amount of substrate (biomass or intermediate product), product inhibition and a non-ideal pH. Assuming that the above growth curve is to a certain extent point-symmetrical, the highest growth speed, taken as the derivative of the concentration in time, then lies at a concentration of micro-organisms of 50%. Assuming that the newly supplied biomass will have a comparatively low concentration of micro-organisms, this means that, if half the exhausted biomass is mixed with newly supplied fresh biomass, the condition for maximizing the growth speed of the micro-organisms is maintained, this considerably accelerating the conversion process. A semi-continuous conversion process is therefore obtained in this way. A wider range, in which replacement of exhausted biomass by fresh biomass will be optimal, will generally be applied in practice. A maximum of 95%, and preferably between 30 and 80%, of the intermediate product present in the first compartment is preferably displaced to the second compartment during step I). The same reasoning and the same values can be applied for replacing residual flows in the second compartment by intermediate product still to be fermented. In order to enable optimizing of the fermenting capacity it is generally more advantageous to apply a plurality of compartments (bioreactors), preferably connected in series, instead of a single compartment, wherein both the acidogenesis and the methanogenesis can be performed simultaneously, though substantially in different compartments. The displacement of at least a part of the intermediate product from the first compartment to the second compartment as according to step I) preferably takes place after detection of a pH progression during step F), this as already stated indicating a decrease in the acid production of at least the fermentative micro-organisms, showing that a substantial part of the biomass has been converted into one or more intermediate products formed as for instance carbon dioxide, hydrogen and/or acetate. The pH progression detected during step F) may be a rise in pH as well as a fall in pH, depending on the moment the base is administered. Particularly in the case dosage of the base to be administered takes place after detecting of the pH value (and the pH progression) as according to step F), a (decreasing) fall in pH per unit of time will then indicate a reduced need for base. In the case the dosage and administering of the base takes place simultaneously with detection of the pH value (and the pH progression) as according to step F), an (increasing) rise in pH per unit of time will indicate a reduced need for base. In a preferred embodiment the pH measurement will form part of a control circuit with which the dosage of the quantity of base to be administered per unit of time and/or the moment at which the biomass has to be displaced can be determined.

The temperature during step A) preferably lies between 20°C and 80°C, more preferably between 30°C and 50°C. This temperature is generally optimal for the acidogenic (fermentative) micro-organisms and acetogenic micro-organisms for the purpose of partial fermentation of the biomass. A temperature lower than 20°C will generally result in a substantially lower biological activity of the relevant micro-organisms, while a higher temperature can cause damage to the micro-organisms, which will also reduce the biological activity. Also known are acidogenic micro-organisms which are biologically active outside the above stated temperature range. The same temperature range can be assumed for the purpose of converting the formed intermediate products to methane by means of the methanogenic micro-organisms. However, diverse methanogenic micro-organisms display biological activity in a wider temperature range, i.e. from 0°C to 110°C, whereby there is more freedom in the temperature which can be applied during step B). It is otherwise found in practice that particularly methanogenic bacteria function optimally in a quite precise temperature range (typically about 3°C around optimum temperature), while the other bacteria, in particular the hydrolytic and acidogenic bacteria, show a significant conversion in a much wider temperature range.

In order to enable relatively precise control of the fermentation process, it is advantageous that the temperature of the biomass during step A) and the intermediate product during step B) is detected by means of one or more thermometers. In order to be able to prevent the temperature of the biomass falling during step A) below the temperature at which the micro-organisms display a (significantly) reduced or even no further activity, and in order to be able to guarantee that the temperature is held substantially constant and at an optimum value, it is generally advantageous when the biomass during step A) and the intermediate product during step B) are actively heated. Heating of the biomass can be realized in diverse ways, although will generally be realized by applying one or more heating elements. It is also possible to envisage heating the biomass, and optionally the intermediate product, by means of irradiation of the biomass and the intermediate product. The heat released during the conversion process is preferably employed to hold the biomass and/or the intermediate products at temperature.

In an alternative preferred embodiment only the biomass is heated during step B), wherein the temperature is held within several degrees or less of the temperature setting. In this case the biomass is heated during step A) only when it threatens to fall below a minimum temperature at which there is danger of low activity or even freezing.

The number of micro-organisms required for an efficient fermentation according to step A) or step B) preferably lies between 10⁷ and 10¹² per gram biomass (including water). An article by Jiunn-Jyi Lay, Yu-You Li, Tatsuua Noike, "Developments of Bacterial Population and Methanogenic Activity in a Laboratory-Scale Landfill Bioreactor", Wat. Res. 32 (1998) 3673-3679 refers to a concentration of acetogenic micro-organisms of 2.45∂10⁸ per gram solid and a concentration of methanogenic micro-organisms van 8.5∂10⁸ per gram solid. The mass of the micro-organisms required for an efficient fermentation according to step A) and step B) preferably amounts to a maximum of 15% by mass, and more preferably 5% by mass. It is noted in this respect that the mass of the micro-organisms in a ruminant, a mammal which likewise converts biomass to methane, generally amounts to about 1.5% by mass.

Before and/or during step A) the biomass is preferably finely ground, chopped, cut or otherwise crushed in order to enable acceleration of the conversion process according to the invention. In a preferred embodiment the biomass is kept in motion during step A) and the intermediate product is kept in motion during step B). By keeping the biomass and the intermediate product in motion an intensive mixing of the biomass and the intermediate product with the micro-organisms can be achieved, this enhancing the efficiency of the fermentation process. A homogenous temperature distribution, base dispersal and/or acid dispersal in the biomass and the intermediate product are also important for the purpose. The biomass and the intermediate product can here be kept in motion continuously, although it is also possible to envisage displacement of the biomass and the intermediate product taking place for instance semi-continuously.

Performing steps A) and B) of the method according to the invention will generally result in a mixture of methane and a residual fraction, wherein the residual fraction will generally comprise non-converted biomass, non-converted intermediate product, water, leftover salts and a remaining fraction. The method preferably also comprises step J), comprising of separating the methane from the residual fraction during or after forming of methane as according to step B). The residual fraction of non-fermented or fully fermented materials is usually also referred to as digestate. The digestate can further be usefully applied as manure. Also formed in addition to methane (about 66%) when the method according to the invention is applied are carbon dioxide (about 33%) and traces of other gases such as hydrogen, dihydrogen sulphide and water (vapour). The whole gas mixture is also usually referred to as biogas. The biogas can generally be separated relatively easily from the digestate, for instance by collecting the biogas. The methane will then generally have to be desulphurized, preferably by adding (atmospheric) oxygen to the biogas, whereby the dihydrogen sulphide can be biologically converted, and will also have to be dehumidified, preferably by means of a condensation process, before the methane is transported and usefully employed. Instead of atmospheric oxygen, oxygen possibly formed in the electrochemical cell can also be applied in the desulphurizing step.

The invention also relates to a device for converting biomass to methane, in particular by applying the method according to the invention, comprising: at least one first compartment for receiving biomass to be converted into at least one intermediate product, at least one base-delivering source connected to the first compartment, at least one second compartment for receiving the at least one intermediate product to be converted to methane and at least one acid-delivering source connected to the second compartment, wherein the acid-delivering source and the base-delivering source form part of at least one electrochemical cell. Advantages of applying a device according to the invention have already been described at length in the foregoing. The first compartment and/or the second compartment preferably take a substantially medium-tight form, in order to prevent as far as possible the entry of atmospheric oxygen into the compartments, since oxygen has an adverse effect on the (anaerobic) fermentation process.

By applying one or more electrochemical cells a predefined quantity of a predefined base or acid can be generated by means of a redox reaction. Equal molar quantities of base and acid can moreover be added in this way to the first compartment and the second compartment, this giving the fermentation process to be performed by means of the device neutral acidity. In a particular preferred embodiment the electrochemical cell is formed by an electrolysis cell. Advantages of applying an electrolysis cell have already been described in the foregoing. The electrochemical cell, in particular the electrolysis cell, preferably comprises two mutually coupled electrochemical half cells.

A base-generating half cell is preferably arranged integrated into the first compartment, and/or an acid-generating half cell is preferably arranged integrated into the second compartment. In an alternative preferred embodiment the at least one electrochemical cell is placed at a distance from (though in the vicinity of) the first compartment and the second compartment so as to enable improved control of the electrochemical process. Further advantages of the two preferred embodiments have already been described in the foregoing.

An optionally applicable additional base-delivering source and an optionally applicable additional acid-delivering source can for instance be formed by respectively a first storage container and a second storage container for storing respectively the base and the acid. It is however also possible to envisage the base-delivering source not being primarily adapted for storage of the base but for generating and subsequently delivering the base. The same can apply for the acid-delivering source.

The first compartment and the second compartment can optionally be integrated with each other and as such form one compartment. The shared compartment here forms a bioreactor which in a preferred embodiment is embodied as a (bio)plug flow reactor. In an alternative preferred embodiment the first compartment and the second compartment are mutually coupled in series, wherein a closing valve is more preferably arranged between the first compartment and the second compartment. After conversion of biomass into at least one intermediate product chosen from the group of: acetate, hydrogen and carbon dioxide, in the first compartment, the closing valve is opened and at least a part of the content of the first compartment is displaced to the second compartment, where the at least one intermediate product will be converted into at least methane. The closing valve will then be reclosed, after which the first compartment can once again be filled with fresh (aqueous) biomass still to be converted.

In a preferred embodiment the first compartment is provided with at least one first acidity meter for detecting the pH in the first compartment. Advantages of applying an acidity meter in the compartment adapted to perform step A) of the method according to the invention have already been described in the foregoing. In a particular preferred embodiment the device is provided with a control unit, in particular a control unit adapted to determine, on the basis of the pH detected by the first acidity meter, the quantity of formed intermediate product and/or the quantity of base to be administered and/or the quantity of administered base. Detection of the acidity progression through time can give an indication of the activity of the fermentation of the biomass into the at least one intermediate product, and therefore about the quantity of formed intermediate product. If a (substantial) decrease in acid production or even rise in pH is detected in the first compartment, this then implies that the above stated fermentation has been substantially realized, whereby the control unit can preferably actuate, in particular open, the closing valve in order to enable displacement of at least part of the content of the first compartment to the second compartment. In the second compartment the pH is preferably also measured by applying at least one second acidity meter so as to be able to monitor the fermentation process in the second compartment. The control unit is preferably (also) adapted to determine, on the basis of the pH detected by the second acidity meter and the quantity of acid already administered, the quantity of methane formed and/or the quantity of acid to be administered and/or the quantity of acid already administered. The control unit is more preferably further adapted to open the second compartment, in particular after detection of a declining acid consumption, for the purpose of enabling removal of at least a part of the digestate from the second compartment. The second compartment will generally also be provided for this purpose with a closing valve which can be actuated by the control unit. The detected pH progression may involve a rise in pH as well as a fall in pH, depending on the moment the acid is administered. When dosing of the acid to be administered takes place particularly after detection of the pH value (and the pH progression), a (decreasing) rise in pH per unit of time will then indicate a reduced need for acid. When dosing and administering of the acid takes place in particular simultaneously with detection of the pH value (and the pH progression), an (increasing) fall in pH per unit of time will then indicate a reduced need for acid.

In a preferred embodiment the first compartment and/or the second compartment is provided with heating means to enable heating of respectively the biomass and the intermediate product. The biomass and the intermediate product can in this way be held at an ideal temperature of between 20°C and 80°C, more preferably between 30°C and 50°C. In order to enable detection of the temperature in the first compartment and/or the second compartment, the relevant compartment is preferably provided with at least one thermometer. The control unit is preferably adapted to actuate the heating means subject to the temperature detected by the thermometer in order to enable the temperature in the first and/or the second compartment to be held at an optimum value.

The first compartment and/or the second compartment is preferably provided with transport means in order to keep respectively the biomass and the intermediate product in motion. Sufficient mixing of the biomass and the intermediate product with the micro-organisms can in this way be guaranteed, which will generally enhance the fermentation process.

In a preferred embodiment there are formed during step A) acid and one or more-intermediate products in which substrates are already present which can be immediately converted by methanogenic bacteria, for instance acetate. Addition of one or more acidic intermediate products to step B) immediately decreases the pH during step B), enhances formation of methane, this subsequently resulting in an increase in pH, and provides the option of completing hydrolysis and acidogenesis not completed during step A).

In a preferred embodiment of the quantity of biomass and/or intermediate products to be added as a consequence of step A) is determined on the basis of the pH and volume of biomass during step B) in order to prevent too low a pH and associated inhibition of methane-formation.

In a preferred embodiment it is also possible to allow the formed biogas above the biomass and liquid to flow away during step B) by means of a valve, whereby the pressure of carbon dioxide above the biomass flows away, thereby reducing the partial pressure of CO2, in order to remove dissolved CO2 from the solution. The increase in pH which occurs here can be used as pH monitoring mechanism. In a further preferred embodiment the partial CO2 pressure can be reduced by replacing the formed biogas with an oxygen-free inert gas, such as for instance nitrogen gas or methane. In yet another embodiment the partial CO2 pressure can be reduced by applying a vacuum pump.

The invention will be elucidated with reference to non-limitative exemplary embodiments shown in the following figures. Herein:
figure 1 shows a schematic representation of the fermentation process for converting biomass to methane,
figure 2 shows a graphic representation of the acidogenic activity of micro-organisms as a function of the acidity,
figure 3a is a graphic representation of the methanogenic activity of Methanosarcina Barkeri and Methanosarcina Hungatei as a function of the acidity,
figure 3b is a graphic representation of the methanogenic activity of Methanosarcina Soehngenii and Methanosarcina Mazei as a function of the acidity,
figure 4 is a schematic representation of the conversion of biomass to methane in accordance with the method according to the invention,
figure 5 shows a schematic representation of a first preferred embodiment of a device according to the invention,
figure 6 shows a schematic representation of a second preferred embodiment of a device according to the invention,
figure 7 is a schematic representation of a third preferred embodiment of a device according to the invention,
figure 8 is a schematic representation of a fourth preferred embodiment of a device according to the invention,
figure 9 is a schematic representation of a fifth preferred embodiment of a device according to the invention,
figure 10 is a schematic representation of a sixth preferred embodiment of a device according to the invention, and
figure 11 is a schematic representation of a seventh preferred embodiment of a device according to the invention.

Figure 1 shows a schematic representation of the fermentation process for converting biomass to methane. This fermentation process can be divided into the following stages which can be performed successively as well as simultaneously: hydrolysis (I), acidogenesis (or fermentation) (II), acetogenesis (III) and methanogenesis (IV).

During the hydrolysis (I) complex, undissolved biopolymers (fats (∀), proteins (∋) and carbohydrates (()) forming part of the biomass are converted into less complex, dissolved compounds (amino acids and sugars (*) and fatty acids and alcohols (y) such as for instance glyceryl) due to the action of extracellular enzymes formed by fermentative micro-organisms.

Acidogenesis or fermentation (II) is defined as a microbiological acid-producing degradation process wherein organic compounds can serve both as electron donor and electron acceptor. Dissolved complex organic compounds are here converted into mainly volatile fatty acids (-COOH). The acid formation is brought about by a large and highly varied group of fermentative bacteria. Examples hereof are the groups of Clostridium and Bacteroides. The great majority of the fermentative bacteria are obligately anaerobic (obligately oxygen-free bacteria), although a part of the population generally consists of optionally anaerobic bacteria. This latter group is able to metabolically convert oxygen remnants present in the biomass. The obligately anaerobic bacteria, including also the methanogens, are in fact hereby protected against oxygen inhibition.

The formed end products in the acidogenesis depend particularly on the genus of bacteria and the process conditions (pH, temperature, redox potential) in addition to the nature of the fermenting device. In two-stage fermentation (wherein biomass is converted in a first compartment to one or more intermediate products, and wherein the intermediate products are converted in a second step to methane) a mixture of, among others, butyrate, acetate, propionate, ethanol (-OH), CO₂ and H₂ is for instance formed, while in a single-stage fermentation (wherein biomass is converted in one shared compartment to methane) acetate, H₂ and CO₂ are the most important products of the fermentative bacteria. The most important products of the fermentative (acidogenic) bacteria are converted by the acetogenic bacteria into acetate, hydrogen and carbon dioxide. Examples of acetogenic reactions in the context of this patent specification are:
CH₃CHOHCOO⁻ + 2 H₂O → CH₃COO⁻ + HCO₃⁻ + H⁺ + 2 H₂
CH₃CH₂OH + H₂O → CH₃COO⁻ + H⁺ + 2 H₂
CH₃CH₂CH₂COO⁻ + 2 H₂O → 2 CH₃COO⁻ + H⁺ + 2 H₂
CH₃CH₂COO⁻ + 3 H₂O → CH₃COO⁻ + HCO₃⁻ + H⁺ + 3 H₂
4 CH₃OH + 2 CO₂ → 3 CH₃COOH + 2 H₂O
2 HCO3⁻ + 4 H₂ + H⁺ → CH₃COO⁻ + 4 H₂O

The above stated processes all have a negative Gibbs energy and will thus proceed spontaneously. However, the conversion of for instance ethanol, butyrate and propionate will not take place under standard conditions at a high partial pressure of hydrogen because of a positive Gibbs energy. A low hydrogen concentration can favourably affect such reactions. It is favourable here when the partial pressure of hydrogen does not lie above 10⁴ atmosphere. On average this partial pressure will generally lie around 10⁻⁶ atmosphere. A low hydrogen pressure is guaranteed when the formed hydrogen is removed sufficiently quickly by hydrogen-consuming bacteria. It is therefore generally important that both the hydrogen-producing bacteria and the hydrogen-consuming bacteria function as well as possible.

The methanogenesis (IVa; IVb) is the final step in the biogas production process. This step is effected mainly by two groups of bacteria, i.e. by the hydrogenotrophic and the acetotrophic methanogens. The hydrogenotrophic methanogens are adapted to convert hydrogen and carbon dioxide to methane (see arrow IVb) in accordance with the following reaction:

4 H₂ + HCO₃⁻ + H⁺ → CH₄ + 3 H₂O

These hydrogenotrophic methanogens are generally responsible for about 30% of the biogas which is produced in a normally operating anaerobic fermentation device and of which methane forms part. Conversely, the acetotrophic methanogens are generally responsible for about 70% of the total produced biogas. These latter methanogens are usually also referred to as the acetate-decomposing bacteria because they decompose the carboxyl group of acetate while forming carbon dioxide (CO₂) and methane (CH₄) (see arrow IVa) in accordance with the following reaction:

CH₃COO⁻ + H⁺ → CH₄ + CO₂

As already stated however, methane can also be formed microbiologically in other ways. The formed methane can be separated from the formed carbon dioxide via conventional methods.

Figure 2 shows a graphic representation of the acidogenic activity of microorganisms as a function of the acidity. The activity shown in figure 2 relates to the overall activity of acidogenic micro-organisms. Figure 2 shows clearly that the acidogenic micro-organisms have an optimum activity at an acidity (pH) of between 5 and 7.

Figure 3a shows a graphic representation of the methanogenic activity of Methanosarcina Barkeri and Methanosarcina Hungatei as a function of the acidity, and figure 3b shows a graphic representation of the methanogenic activity of Methanothrix Soehngenii and Methanosarcina Mazei as a function of the acidity. It can clearly be seen that the biological activity of these methanogenic micro-organisms decreases substantially at an acidity higher than 7.5. It is also expected that the activity will also decrease at an acidity lower than 6.5, whereby these methanogenic micro-organisms will be optimally active at an acidity between 6.5 and 7.5.

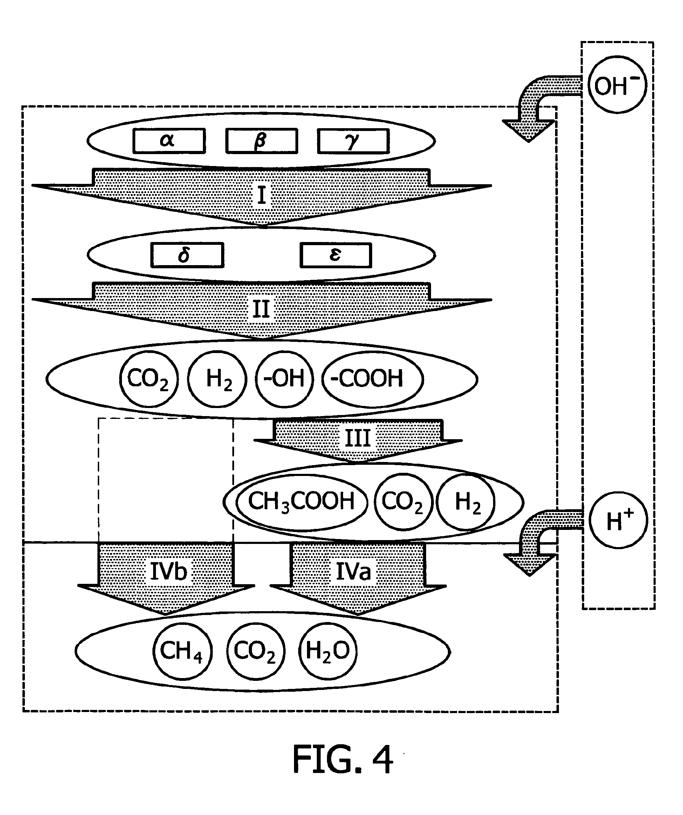

Figure 4 shows a schematic representation of the conversion of biomass to methane in accordance with the method according to the invention. The fermentation process shown in figure 4 is identical to the fermentation process shown in figure 1, with the difference that a base (OH⁻) is added to the biomass to enable optimization of the hydrolysis, and the acidogenesis and optionally the acetogenesis. The function of the base is to keep the acidity sufficiently high (between 5 and 7) to enable optimizing of the biological activity of the micro-organisms. Before and during the methanogenesis an acid (H⁺) is added in a relatively complex reaction mechanism which results in the formation of methane.

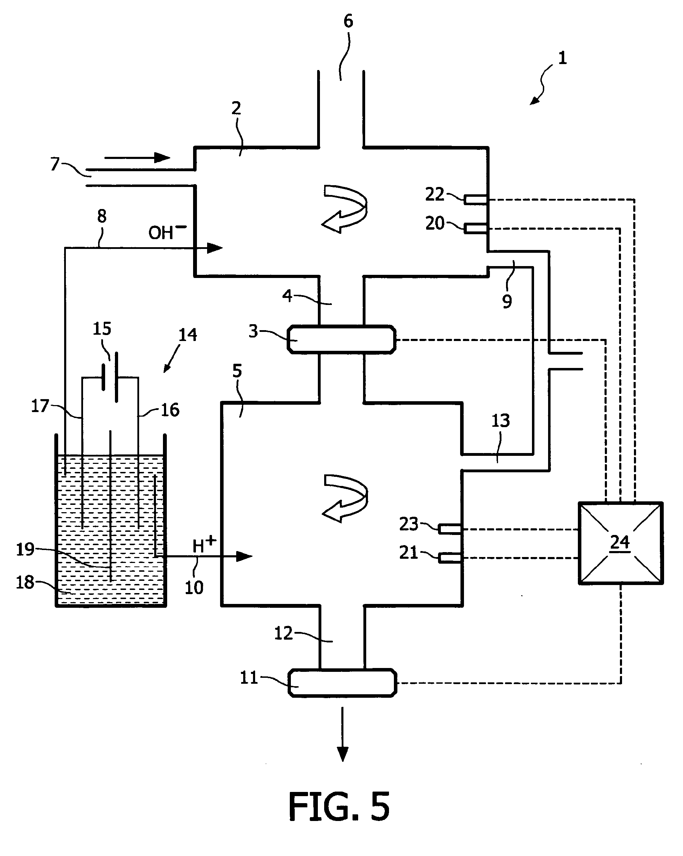

Figure 5 shows a schematic representation of a first preferred embodiment of a device 1 according to the invention. Device 1 comprises a first compartment 2 and a second compartment 5 connected to first compartment 2 via an intermediate conduit 4 closable by means of a closing valve 3. First compartment 2 is adapted to allow hydrolysis, acidogenesis and optionally (part of) the acetogenesis to take place for the purpose of forming the intermediate products carbon dioxide, hydrogen and acetate. Second compartment 5 is adapted mainly to allow the methanogenesis to take place, wherein the formed intermediate products are converted into (among others) methane, carbon dioxide and water. First compartment 2 is provided with a first inlet 6 for biomass, a second inlet 7 for water for wetting the biomass, and a third inlet 8 for base for the purpose of preventing considerable acidification of the biomass. First compartment 2 is also provided with an outlet 9 for biogas which is possibly formed in first compartment 2 and which, in addition to methane, can also comprise carbon dioxide, hydrogen gas, hydrogen sulphide, nitrogen and other gases. Second compartment 5 is provided with an inlet 10 for acid, a first outlet 12 for digestate closable by means of a closing valve 11, and a second outlet 13 for formed biogas. The base and the acid are formed using an electrolytic cell 14 which is shown schematically in figure 5. Electrolytic cell 14 comprises a power source 15, this power source 15 being provided with a positive electrode 16 and a negative electrode 17. Both electrodes 16, 17 are arranged in an aqueous liquid 18. Also arranged in liquid 18 is a conductive separating wall 19 (shown schematically), such as for instance a membrane, which substantially separates the anodic and cathodic compartments from each other. Through the agency of power source 15 the water will decompose around electrodes 16, 17 in accordance with the following reaction equations:
Negative electrode: 4 H₂O + 4e⁻ ) 4 OH⁻ + 2H₂
Positive electrode: 2 H₂O ) _{O2} + 4 H⁺ + 4 e⁻

The formed base (OH⁻) and the acid (H⁺) are formed during the reaction in equivalent molar quantities and are supplied as such to first compartment 2 and second compartment 5, whereby the overall conversion process preferably retains neutral acidity. Salt can optionally be added to aqueous liquid 18 to enable the conductivity of the liquid to be improved. A buffer can optionally (also) be added to the aqueous liquid in order to enable improved regulation of the final acidity of the biomass and/or the intermediate product. Stirring means and cutting means (not shown), such as for instance a chopping element, are arranged in both first compartment 2 and second compartment 5 for the purpose of keeping in motion and finely cutting the biomass and subsequently the intermediate products and the digestate (compost). Both first compartment 2 and second compartment 5 are provided with an acidity meter 20, 21 and a thermometer 22, 23. Acidity meters 20, 21, thermometers 22, 23 and both closing valves 3, 11 are coupled to a control unit 24. Control unit 24 is adapted to process the acidity and temperature detected in each compartment 2, 5 and, subject to these, to also supply respectively base and acid via inlets 8, 10, as well as to selectively actuate (open or close) the respective closing valves 3, 11 and to feed fresh biomass via first inlet 6. Device 1 is optionally provided with heating means (not shown) to enable heating of first compartment 2 and/or second compartment 5, The heating means are here preferably coupled to control unit 24, and are more preferably actuated by control unit 24 on the basis of the temperatures detected by thermometers 22, 23. An elaboration of the actuation of device 1 by control unit 24 in addition to further advantages of device 1 have already been described at length in the foregoing description.

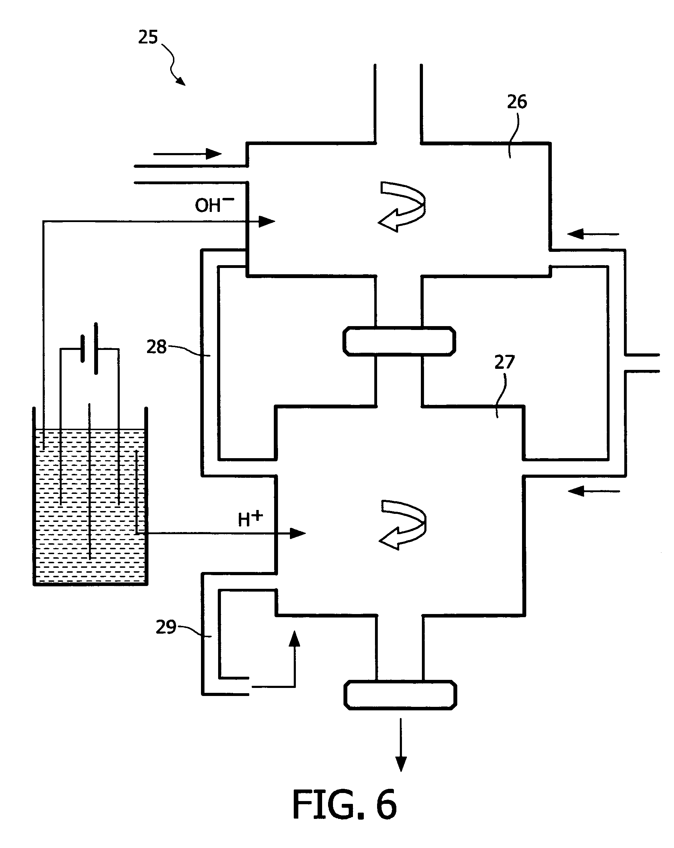

Figure 6 shows a schematic representation of a second preferred embodiment of a device 25 according to the invention. Device 25 is structurally similar to device 1 shown in figure 1, wherein the fermentation is also effected in a plurality of compartments 26, 27 connected in series, but wherein device 25 is provided with a first return line 28 for guiding back to first compartment 26 a part of the content of second compartment 27, formed mainly by a mixture of biomass, digestate, intermediate product and micro-organisms. Device 25 is also provided with a second return line 29 for guiding a part of the digestate already provided with micro-organisms from second compartment 27 back into second compartment 27. By means of return lines 28, 29 the population of micro-organisms in compartments 26, 27 can be brought relatively quickly to an (initial) level from which relatively rapid growth of micro-organisms can take place, this enhancing the speed and efficiency of the fermentation process. Return lines 28, 29 are preferably provided with a pump (not shown) for displacing the biomass.

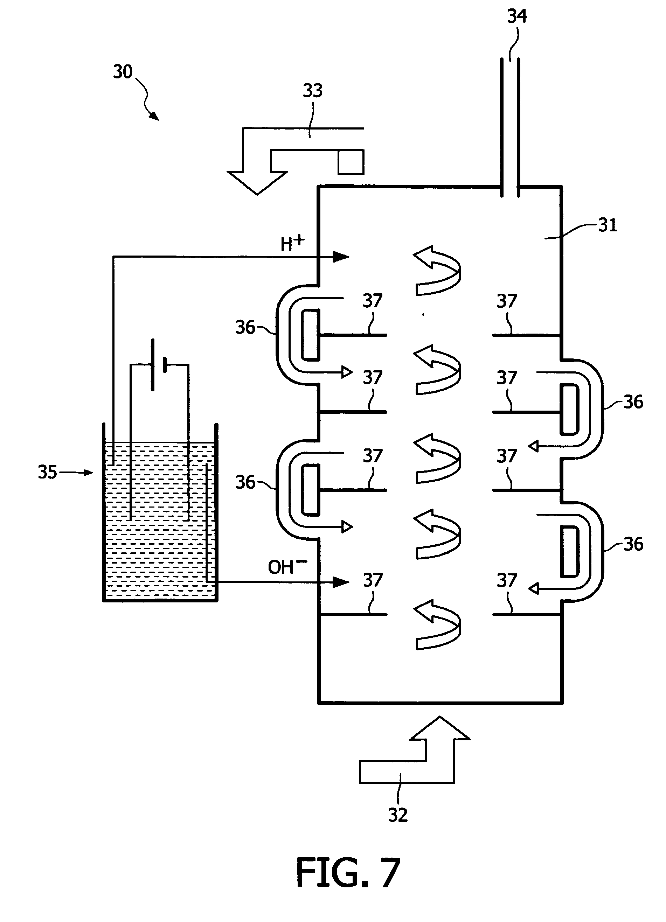

Figure 7 shows a schematic representation of a third preferred embodiment of a device 30 according to the invention. Device 30 comprises a vertically oriented bioreactor 31 which in fact consists of one compartment. The biomass to be converted to methane is introduced via an inlet 32 into a bottom part of bioreactor 31 and displaced in upward direction by means of an Archimedes screw (not shown). Instead of applying an Archimedes screw, the biomass can also be displaced in other manner in bioreactor 31, for instance by exerting pressure on the biomass. At a top side the digestate, formed mainly by moist, exhausted biomass, is discharged via a first outlet 33, and the biogas formed in bioreactor 31 is collected via a second (gas) outlet 34. Device 30 also comprises an electrolytic cell 35 which is connected to bioreactor 31 such that base (OH⁻) is guided into a lower part of bioreactor 31 and acid (H⁺) is guided into an upper part of bioreactor 31. Advantages of adding base and acid location-selectively to bioreactor 31 have already been described in the foregoing. Device 30 further comprises different return lines 36 for guiding a fraction of the content of bioreactor 31 back to a lower-lying part in bioreactor 31 in order to enable improved mixing in bioreactor 31 and optimizing of the micro-organism management in bioreactor 31. Each return line 36 will be provided for this purpose with a pump (not shown). Diverse baffles 37 are arranged in bioreactor 31 in order to limit vertical mixing in bioreactor 31 to some extent.

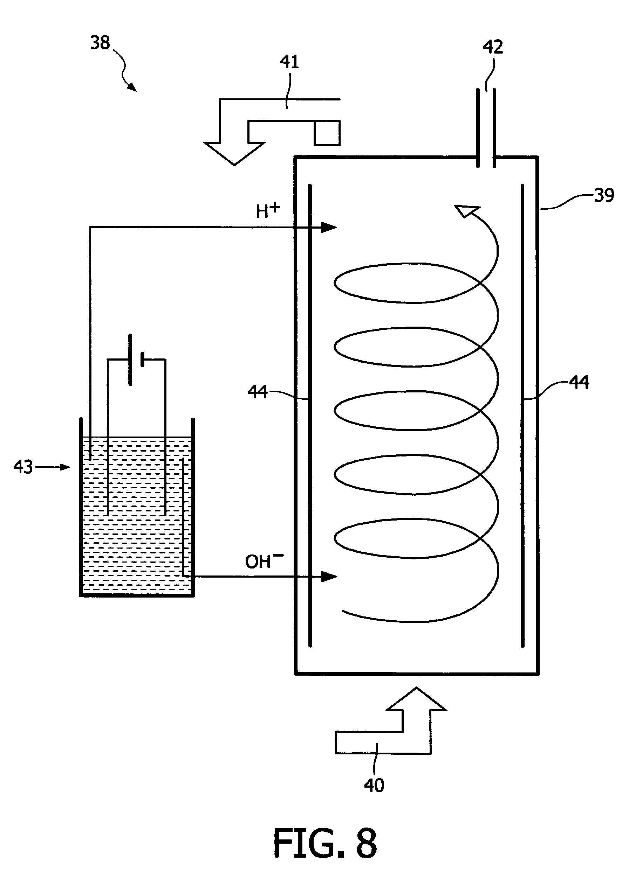

Figure 8 shows a schematic representation of a fourth preferred embodiment of a device 38 according to the invention. Device 38 comprises a vertically oriented bioreactor 39 consisting of one shared compartment. The orientation of bioreactor 39 is arbitrary, wherein it is for instance also possible to envisage the bioreactor 39 being disposed in a horizontal or diagonal orientation. A biomass to be converted into biogas is introduced via an inlet 40 into a bottom part of bioreactor 39 and displaced in upward direction by means of an Archimedes screw (not shown). On a top side the digestate, formed mainly by moist, exhausted biomass, is discharged via a first outlet 41 and the biogas formed in bioreactor 39 is collected via a second (gas) outlet 42. Device 38 also comprises an electrolytic cell 43 which is connected to bioreactor 39 such that base (OH⁻) is guided into a lower part of bioreactor 39 and acid (H⁺) is guided into an upper part of bioreactor 39. Advantages of adding base and acid location-selectively to bioreactor 39 have already been described in the foregoing. Device 38 further comprises a plurality of structures 44 adapted as adhesion surface and growth surface for micro-organisms. The efficiency of the fermentation process can be improved by stimulating the growth of micro-organisms in bioreactor 39 on structures 44.

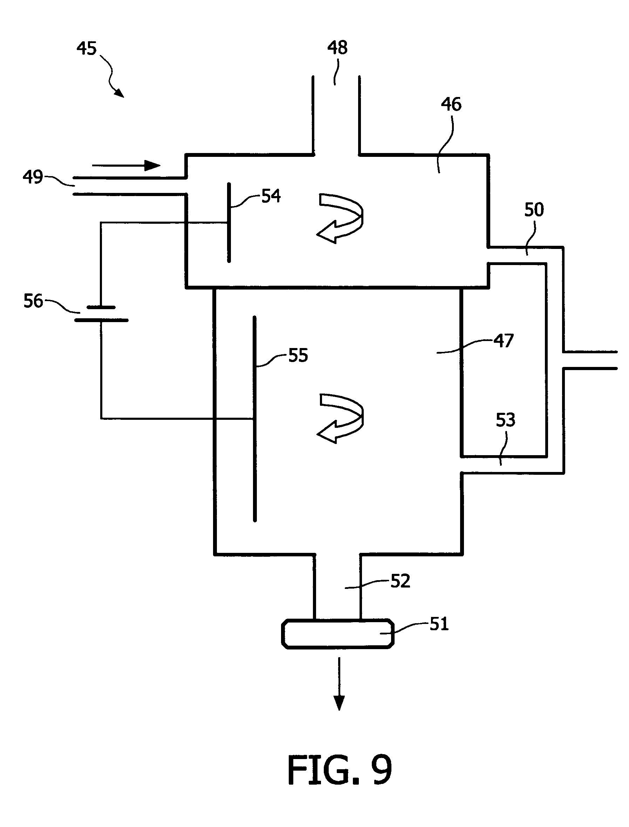

Figure 9 shows a schematic representation of a fifth preferred embodiment of a device 45 according to the invention. Device 45 comprises a first compartment 46 and a second compartment 47 connecting directly onto first compartment 46. First compartment 46 is adapted to allow the hydrolysis, acidogenesis and optionally (part of) the acetogenesis to take place for the purpose of forming the intermediate products carbon dioxide, hydrogen and acetate. Second compartment 47 is adapted mainly to allow the methanogenesis and optionally (part of) the acetogenesis to take place, wherein the formed intermediate products are converted into methane, carbon dioxide and water. First compartment 46 is provided with a first inlet 48 for biomass, a second inlet 49 for water for wetting the biomass, and an outlet 50 for biogas possibly formed in first compartment 46. Second compartment 47 is provided with a first outlet 52 for digestate closable by means of a closing valve 51, and a second outlet 53 for formed biogas. It is particularly advantageous according to the invention to add base and acid location-selectively in order to optimize the fermentation process. In this exemplary embodiment the base and the acid are generated respectively in first compartment 46 and second compartment 47. For this purpose device 45 comprises a negative electrode 54 arranged in first compartment 46 and a positive electrode 55 arranged in second compartment 47, these electrodes 54, 55 being connected to a power source 56. By introducing electrodes 54, 55 into compartments 46, 47 each compartment 46, 47 in fact forms an electrolytic half cell for the purpose of generating respectively base and acid from the water supplied via second inlet 49 and the water already present in the biomass supplied via first inlet 48.

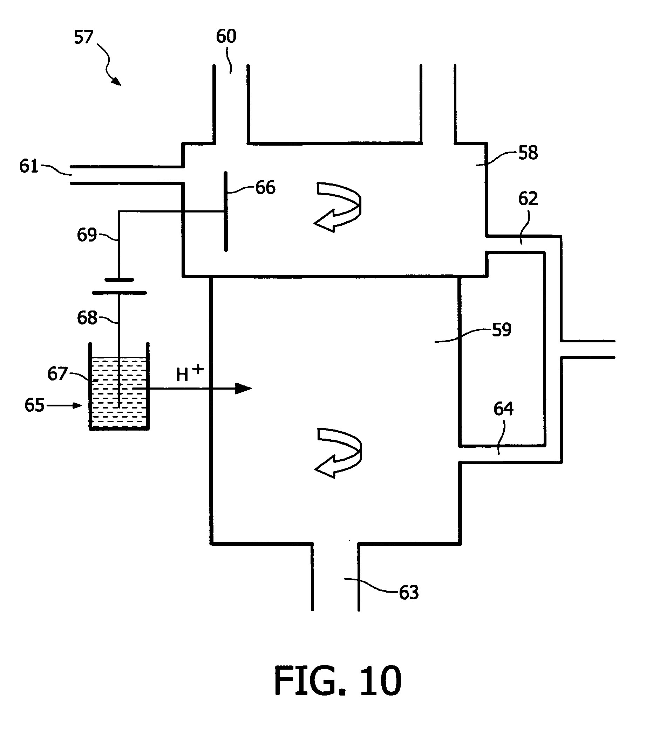

Figure 10 shows a schematic representation of a sixth preferred embodiment of a device 57 according to the invention. Device 57 comprises a first compartment 58 and a second compartment 59 connecting directly onto first compartment 58. First compartment 58 is adapted to allow hydrolysis, acidogenesis and optionally (part of) the acetogenesis to take place for the purpose of forming the intermediate products carbon dioxide, hydrogen, acetate and/or possible alternative intermediate products. Second compartment 59 is adapted mainly to allow the methanogenesis and optionally (part of) the acetogenesis to take place, wherein the formed intermediate products are converted into at least methane, carbon dioxide and water. First compartment 58 is provided with a first inlet 60 for biomass and a second inlet 61 for water for wetting the biomass, and an outlet 62 for biogas possibly formed in first compartment 58. First compartment 58 is provided with cutting means to enable fine cutting and optionally keeping in motion of the biomass in first compartment 58. Second compartment 59 comprises an outlet 63 for digestate and an outlet 64 for biogas. As shown in this figure, device 57 comprises a first electrolytic half cell 65 positioned at a distance from compartments 58, 59, and a second electrolytic half cell 66 which is arranged integrated into first compartment 58. The first half cell 65 is provided with an aqueous solution 67 in which an anode 68 is arranged, whereby oxygen (O₂) and acid (H⁺) will be formed in first half cell 65. The acid will subsequently be guided to second compartment 59, while the formed oxygen, which has an adverse effect on the microbiological conversion of biomass to methane, is optionally collected and discharged. Second half cell 66 is provided with a cathode 69 which is arranged integrated into first compartment 58, whereby hydrogen (H₂) and base (OH') will be formed in the first compartment. The addition of base to first compartment 58 is advantageous as described at length in the foregoing. The addition of hydrogen likewise enhances efficient conversion of biomass to methane, all the more so in that hydrogen forms one of the starting products enabling the formation of methane. Also arranged in compartments 58, 59 is a conductive salt bridge (not shown) for the purpose of electrical connection of half cells 65, 66 to each other.

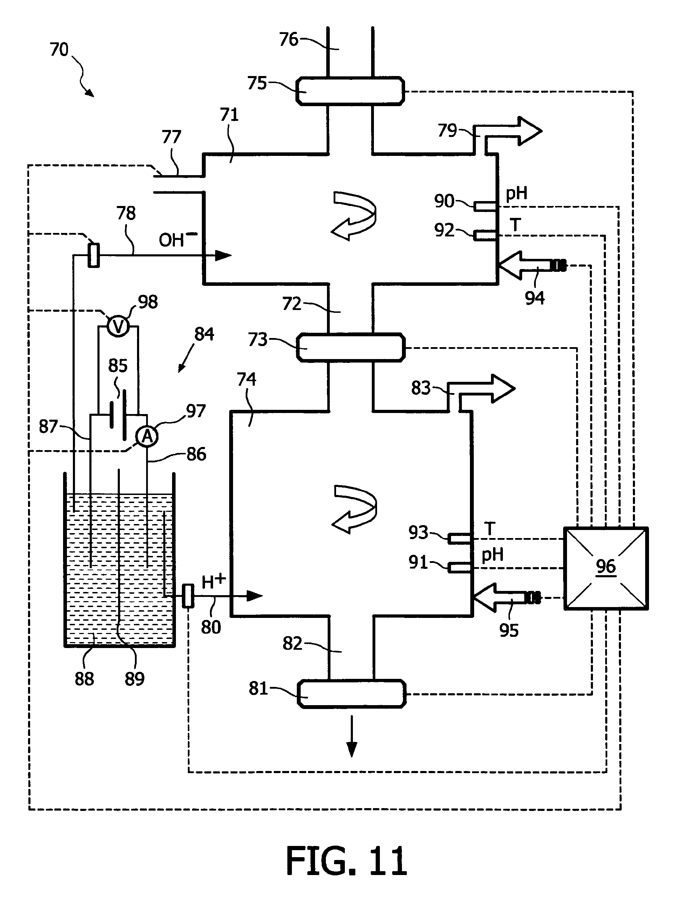

Figure 11 shows a schematic representation of a seventh preferred embodiment of a device 70 according to the invention. Device 70 comprises a first compartment 71 and a second compartment 74 connected to first compartment 71 via an intermediate conduit 73 closable by means of a closing valve 72. First compartment 71 is adapted to allow the hydrolysis, acidogenesis and optionally (part of) the acetogenesis to take place for the purpose of forming the intermediate products such as for instance carbon dioxide, hydrogen and acetate. Second compartment 74 is adapted mainly to allow the methanogenesis to take place, wherein the formed intermediate products are converted into (among others) methane, carbon dioxide and water. First compartment 71 is provided with a first inlet 76 for biomass closable by a closing valve 75, a second inlet 77 for water for wetting the biomass, and a third inlet 78 for base for the purpose of preventing considerable acidification of the biomass. First compartment 71 is also provided with an outlet 79 for biogas possibly formed in first compartment 71. Second compartment 74 is provided with an inlet 80 for acid, a first outlet 82 for digestate closable by means of a closing valve 81, and a second outlet 83 for formed biogas. The base and the acid are formed using an electrolytic cell 84 which is shown schematically. Electrolytic cell 84 comprises a power source 85, this power source 85 being provided with a positive electrode 86 and a negative electrode 87. Both electrodes 86, 87 are arranged in an aqueous liquid 88. Also arranged in liquid 88 is a conductive separating wall 89, such as for instance a membrane. Through the agency of power source 85 the water will decompose around electrodes 86, 87 in accordance with the following reaction equations:
Negative electrode: 4 H₂O + 4e⁻ ) 4 OH⁻ + 2H₂
Positive electrode: 2 H₂O ) O₂ + 4 H⁺ + 4 e⁻

The formed base (OH⁻) and the acid (H⁺) are formed during the reaction in equivalent molar quantities and are supplied as such to first compartment 71 and second compartment 74, whereby the overall conversion process retains neutral acidity. Stirring means and cutting means (not shown) such as for instance a chopping element, are arranged in both first compartment 71 and second compartment 74 for the purpose of keeping in motion and finely cutting respectively the biomass and the intermediate products and the digestate (compost). Both first compartment 71 and second compartment 74 are provided with an acidity meter 90, 91, a thermometer 92, 93 and heating means 94, 95. Acidity meters 90, 91, thermometers 92, 93, heating means 94, 95, closing valves 72, 75, 81 and optionally the stirring speeds of optionally applied stirring elements (not shown) are controlled by a control unit 96. Control unit 96 is adapted to process the acidity and temperature detected in each compartment 71, 74, the amount of heat supplied and the quantity of base and acid supplied, and, subject hereto, to also selectively actuate (open or close) the respective closing valves 72, 81 and to supply fresh biomass via first inlet 75. In this exemplary embodiment the electrolytic cell 84 is also provided with an ammeter 97 and a voltmeter 98, both of which are also coupled to control unit 96 in order to enable monitoring of the activity of electrolytic cell 84. The control unit will generally be provided with time-recording means to enable observation through time of the progression of process parameters on the basis of which the actuation of device 70 will generally be adjusted. First inlet 76, intermediate conduit 73, inlets 78, 80 and/or outlets 79, 82, 83 can optionally be provided with a flow meter and/or mass meter, which are preferably also coupled to control unit 96 to enable further optimization of the process control.

It will be apparent that the invention is not limited to the exemplary embodiments shown and described here, but that within the scope of the appended claims numerous variants are possible which will be self-evident to the skilled person in this field.

## Claims

1. Method for converting biomass into methane, comprising the steps of:
A) fermenting at least a part of the biomass to form at least one intermediate product, in particular at least one intermediate product chosen from the group of:
hydrogen, carbon dioxide and acetate, carbon monoxide, volatile fatty acids, formate, alcohols, pyruvate, methylamine, dimethylamine, methyl sulphide,
dimethyl sulphide, trimethylamine, methyl mercaptan, and incompletely reduced carbon compounds; and
B) fermenting at least a part of the at least one intermediate product as according to step A) to form at least methane,
wherein the method also comprises the following steps of:
C) generating at least one base and at least one acid using at least one electrochemical cell,
D) adding at least one base generated during step C) to the biomass during step A), and
E) adding at least one acid generated during step C) to the intermediate product during step B).

2. Method as claimed in any of the foregoing claims, **characterized in that** at least one acid generated during step A) is additionally added during step B) to the at least one intermediate product for fermenting.

3. Method as claimed in any of the foregoing claims, **characterized in that** at least one base generated during step B) is additionally added during step A) to the biomass for fermenting.

4. Method as claimed in any of the foregoing claims, **characterized in that** the generating of the base and the acid according to step C) takes place by means of electrolysis of water with forming of H⁺ ions and OH⁻ ions.

5. Method as claimed in any of the foregoing claims, **characterized in that** the method also comprises the following step of:
F) detecting the pH of the biomass during step A).

6. Method as claimed in any of the foregoing claims, **characterized in that** the quantity of base to be administered as according to step D) is at least determined on the basis of the pH detected during step F).

7. Method as claimed in any of the foregoing claims, **characterized in that** the pH lies between 6.5 and 8.0 during step B).

8. Method as claimed in any of the foregoing claims, **characterized in that** the method also comprises the following step of:
G) detecting the pH of the intermediate product during step B).

9. Method as claimed in any of the foregoing claims, **characterized in that** the quantity of acid to be administered as according to step E) is at least determined on the basis of the pH detected during step G).

10. Method as claimed in any of the foregoing claims, **characterized in that** the method also comprises the following steps of:
H) guiding the biomass into a first compartment prior to fermenting of at least a part of the biomass according to step A), and
I) displacing at least a part of the intermediate product from the first compartment to a second compartment prior to fermenting of at least a part of the intermediate product according to step B).

11. Method as claimed in any of the foregoing claims, **characterized in that** the biomass is actively heated during step A) and/or the intermediate product during step B).

12. Method as claimed in any of the foregoing claims, **characterized in that** the biomass is subjected during step A) to a hydrolysis followed by an acidogenesis or an anaerobic oxidation.

13. Method as claimed in claim 12, **characterized in that** the acidogenesis or the anaerobic oxidation is followed by an acetogenesis.

14. Method as claimed in any of the foregoing claims, **characterized in that** the intermediate product is subjected during step B) to a methanogenesis.

15. Device for converting biomass to methane, in particular by applying the method as claimed in any of the claims 1-14, comprising:
- at least one first compartment for receiving biomass to be converted into at least one intermediate product,
- at least one base-delivering source connected to the first compartment,
- at least one second compartment for receiving the at least one intermediate product to be converted to methane,
- at least one acid-delivering source connected to the second compartment, wherein the base-delivering source and the acid-delivering source form part of at least one electrochemical cell.

## Patentansprüche

1. Verfahren zum Umwandeln von Biomasse in Methan, das folgende Schritte umfasst:
A) Fermentieren von mindestens einem Teil der Biomasse, um mindestens ein Zwischenprodukt zu erzeugen, insbesondere mindestens ein Zwischenprodukt, das aus folgender Gruppe ausgewählt wird: Wasserstoff, Kohlendioxid und Acetat, Kohlenmonoxid, flüchtige Fettsäuren, Formiat, Alkohole, Pyruvat, Methylamin, Dimethylamin, Methylsulfid, Dimethylsulfid, Trimethylamin, Methylmercaptan und unvollständig abgebaute Kohlenstoffverbindungen; und
B) Fermentieren von mindestens einem Teil des mindestens einen Zwischenprodukts gemäß Schritt A), um mindestens Methan zu erzeugen,
wobei das Verfahren ferner folgende Schritte umfasst:
C) Erzeugen von mindestens einer Base und mindestens einer Säure unter Verwendung von mindestens einer elektrochemischen Zelle,
D) Hinzufügen von mindestens einer in Schritt C) erzeugten Base zu der Biomasse während Schritt A), und
E) Hinzufügen von mindestens einer in Schritt C) erzeugten Säure zu dem Zwischenprodukt während Schritt B).

2. Verfahren nach einem der vorstehenden Ansprüche, **dadurch gekennzeichnet, dass** mindestens eine in Schritt A) erzeugte Säure in Schritt B) zusätzlich zu dem mindestens einen Zwischenprodukt zum Fermentieren hinzugefügt wird.

3. Verfahren nach einem der vorstehenden Ansprüche, **dadurch gekennzeichnet, dass** mindestens eine in Schritt B) erzeugte Base in Schritt A) zusätzlich zu der Biomasse zum Fermentieren hinzugefügt wird.

4. Verfahren nach einem der vorstehenden Ansprüche, **dadurch gekennzeichnet, dass** das Erzeugen der Base und der Säure gemäß Schritt C) durch Elektrolyse von Wasser mit Bildung von H⁺-Ionen und OH⁻-Ionen erfolgt.

5. Verfahren nach einem der vorstehenden Ansprüche, **dadurch gekennzeichnet, dass** das Verfahren ferner folgenden Schritt umfasst:
F) Ermitteln des pH-Wertes der Biomasse in Schritt A).

6. Verfahren nach einem der vorstehenden Ansprüche, **dadurch gekennzeichnet, dass** die Menge an Base, die gemäß Schritt D) hinzugefügt wird, mindestens auf der Grundlage des in Schritt F) ermittelten pH-Wertes festgelegt wird.

7. Verfahren nach einem der vorstehenden Ansprüche, **dadurch gekennzeichnet, dass** der pH-Wert in Schritt B) zwischen 6,5 und 8,0 liegt.

8. Verfahren nach einem der vorstehenden Ansprüche, **dadurch gekennzeichnet, dass** das Verfahren ferner folgenden Schritt umfasst:
G) Ermitteln des pH-Wertes des Zwischenprodukts in Schritt B).

9. Verfahren nach einem der vorstehenden Ansprüche, **dadurch gekennzeichnet, dass** die Menge an Säure, die gemäß Schritt E) hinzugefügt wird, mindestens auf der Grundlage des in Schritt G) ermittelten pH-Wertes festgelegt wird.

10. Verfahren nach einem der vorstehenden Ansprüche, **dadurch gekennzeichnet, dass** das Verfahren ferner folgende Schritte umfasst:
H) Leiten der Biomasse in eine erste Kammer vor dem Fermentieren von mindestens einem Teil der Biomasse gemäß Schritt A), und
I) Verschieben von mindestens einem Teil des Zwischenprodukts von der ersten Kammer in eine zweite Kammer vor dem Fermentieren von mindestens einem Teil des Zwischenprodukts gemäß Schritt B).

11. Verfahren nach einem der vorstehenden Ansprüche, **dadurch gekennzeichnet, dass** die Biomasse in Schritt A) und/oder das Zwischenprodukt in Schritt B) aktiv erhitzt werden/wird.

12. Verfahren nach einem der vorstehenden Ansprüche, **dadurch gekennzeichnet, dass** die Biomasse in Schritt A) einer Hydrolyse gefolgt von einer Säurebildung oder einer anaeroben Oxidation ausgesetzt wird.

13. Verfahren nach Anspruch 12, **dadurch gekennzeichnet, dass** der Säurebildung oder der anaeroben Oxidation eine Essigsäurebildung folgt.

14. Verfahren nach einem der vorstehenden Ansprüche, **dadurch gekennzeichnet, dass** das Zwischenprodukt in Schritt B) einer Methanbildung ausgesetzt wird.

15. Einheit zum Umwandeln von Biomasse in Methan, insbesondere durch Anwenden des Verfahrens gemäß den Ansprüchen 1 bis 14, wobei die Einheit Folgendes umfasst:
- mindestens eine erste Kammer zum Aufnehmen von Biomasse, die in mindestens ein Zwischenprodukt umgewandelt wird,
- mindestens eine basenliefernde Quelle, die mit der ersten Kammer verbunden ist,
- mindestens eine zweite Kammer zum Aufnehmen des mindestens einen Zwischenprodukts, das in Methan umgewandelt wird,
- mindestens eine säureliefernde Quelle, die mit der zweiten Kammer verbunden ist, wobei die basenliefernde Quelle und die säureliefernde Quelle einen Teil von mindestens einer elektrochemischen Zelle bilden.

## Revendications

1. Procédé pour convertir de la biomasse en méthane, comprenant les étapes consistant :
A) à faire fermenter au moins une partie de la biomasse pour former au moins un produit intermédiaire, en particulier au moins un produit intermédiaire sélectionné dans le groupe constitué de l'hydrogène, du dioxyde de carbone et de l'acétate, du monoxyde de carbone, d'acides gras volatils, du formate, d'alcools, du pyruvate, de la méthylamine, de la diméthylamine, du sulfure de méthyle, du sulfure de diméthyle, de la triméthylamine, du méthylmercaptan et de composés carbonés incomplètement réduits, et
B) à faire fermenter au moins une partie du au moins un produit intermédiaire comme selon l'étape A) pour former au moins du méthane,
étant entendu que le procédé comprend aussi les étapes suivantes consistant :
C) à produire au moins une base et au moins un acide en utilisant au moins une cellule électrochimique ;
D) à ajouter à la biomasse pendant l'étape A) au moins une base produite pendant l'étape C), et
E) à ajouter au produit intermédiaire pendant l'étape B) au moins un acide produit pendant l'étape C).

2. Procédé selon l'une quelconque des revendications précédentes, **caractérisé en ce qu'**au moins un acide produit pendant l'étape A) est ajouté en plus pendant l'étape B) au au moins un produit intermédiaire pour le faire fermenter.

3. Procédé selon l'une quelconque des revendications précédentes, **caractérisé en ce qu'**au moins une base produite pendant l'étape B) est ajoutée en plus pendant l'étape A) à la biomasse pour la faire fermenter.

4. Procédé selon l'une quelconque des revendications précédentes, **caractérisé en ce que** la production de la base et de l'acide selon l'étape C) a lieu au moyen de l'électrolyse d'eau avec formation d'ions H+ et d'ions OH.

5. Procédé selon l'une quelconque des revendications précédentes, **caractérisé en ce que** le procédé comprend aussi l'étape suivante consistant :
F) à détecter le pH de la biomasse pendant l'étape A).

6. Procédé selon l'une quelconque des revendications précédentes, **caractérisé en ce que** la quantité de base à administrer comme selon l'étape D) est au moins déterminée sur la base du pH détecté pendant l'étape F).

7. Procédé selon l'une quelconque des revendications précédentes, **caractérisé en ce que** le pH se situe entre 6,5 et 8,0 pendant l'étape B).

8. Procédé selon l'une quelconque des revendications précédentes, **caractérisé en ce que** le procédé comprend aussi l'étape suivante consistant :
G) à détecter le pH du produit intermédiaire pendant l'étape B).

9. Procédé selon l'une quelconque des revendications précédentes, **caractérisé en ce que** la quantité d'acide à administrer comme selon l'étape E) est au moins déterminée sur la base du pH détecté pendant l'étape G).

10. Procédé selon l'une quelconque des revendications précédentes, **caractérisé en ce que** le procédé comprend aussi les étapes suivantes consistant :
H) à guider la biomasse dans un premier compartiment avant de faire fermenter au moins une partie de la biomasse selon l'étape A), et
I) à déplacer au moins une partie du produit intermédiaire du premier compartiment jusqu'à un second compartiment avant de faire fermenter au moins une partie du produit intermédiaire selon l'étape B).

11. Procédé selon l'une quelconque des revendications précédentes, **caractérisé en ce que** l'on chauffe activement la biomasse pendant l'étape A) et/ou le produit intermédiaire pendant l'étape B).

12. Procédé selon l'une quelconque des revendications précédentes, **caractérisé en ce que** la biomasse est soumise pendant l'étape A) à une hydrolyse suivie d'une acidogenèse ou d'une oxydation anaérobie.

13. Procédé selon la revendication 12, **caractérisé en ce que** l'acidogenèse ou l'oxydation anaérobie est suivie d'une acétogenèse.

14. Procédé selon l'une quelconque des revendications précédentes, **caractérisé en ce que** le produit intermédiaire est soumis pendant l'étape B) à une méthanogenèse.

15. Dispositif pour convertir de la biomasse en méthane, en particulier en appliquant le procédé selon l'une quelconque des revendications 1 14, comprenant :
- au moins un premier compartiment pour recevoir la biomasse à convertir en au moins un produit intermédiaire ;
- au moins une source d'alimentation en base reliée au premier compartiment ;
- au moins un second compartiment pour recevoir le au moins un produit intermédiaire à convertir en méthane ;
- au moins une source d'alimentation en acide reliée au second compartiment, étant entendu que la source d'alimentation en base et la source d'alimentation en acide font partie d'au moins une cellule électrochimique.
